# EUROPEAN PATENT APPLICATION

(11) **EP 4 190 271 A1**
(43) Date of publication of application: **07.06.2023**
(21) Application number: 21212304.6
(22) Date of filing: 03.12.2021
(51) Int. Cl.: A61B 90/00, A61B 1/267, A61B 1/00, G06T 7/13, G06N 3/02, G06T 7/73, A61B 1/31

(54) **ENDOSCOPE IMAGE PROCESSING DEVICE**

(71) Applicant: Ambu A/S, 2750 Ballerup (DK)
(72) Inventor: ALONSO DÍAZ, Alejandro, 2750 Ballerup (DK); GADE, Josefine Dam, 1973 Frederiksberg (DK); JØRGENSEN, Andreas Härstedt, 2610 Rødovre (DK); LASSEN, Lee Herluf Lund, 2760 Måløv (DK); YU, Dana Marie, 2750 Ballerup (DK)
(74) Representative: AWA Denmark A/S

(57) **Abstract**

Disclosed is an image processing device for estimating a quality measure of a endoscopic procedure performed using an endoscope. The endoscope comprising an image capturing device, the image processing device comprising a processing unit operationally connectable to the image capturing device. The processing unit is configured to: obtain a stream of images captured by the image capturing device of the endoscope; process the stream of images to estimate locations of a lumen in the stream of images, and determine a quality measure of the endoscopic procedure based on the estimated locations of the lumen.

## Description

### Field

The present disclosure relates to an image processing device, a display unit, an endoscope system, a method for estimating a quality measure of an endoscopy procedure, and a computer program product.

### Background

Endoscopes are widely used in hospitals for visually examining body cavities and obtaining samples of tissue identified as potentially pathological. An endoscope typically comprises an image capturing device arranged at the distal end of the endoscope either looking forward or to the side. An endoscope is further typically provided with a working channel allowing a medical device such as a gripping device, a suction device, or a catheter to be introduced.

Performing a proper endoscopic procedure where all relevant parts of a body cavity are thoroughly examined for pathological conditions is however a difficult task.

It is therefore important that new medical personnel are properly trained. Training new medical personnel is however expensive and time consuming since an experienced doctor typically must oversee the procedure.

Furthermore, even for experienced medical personnel it may be difficult to assess the quality of their procedures.

A colonoscopy is an example of a complex endoscopic procedure. A medical professional may calculate his / hers Polyp detection rate and / or Ad-enoma detection rate and compare those with expected values. This may provide the medical professional with a general idea of their performance over time. However, an evaluation of the quality of a specific colonoscopy is not possible.

Thus, it remains a problem to provide an improved device / method for allowing medical personnel to train and / or evaluate their performance.

### Summary

According to a first aspect, the present disclosure relates to an image processing device for estimating a quality measure of an endoscopic procedure performed using an endoscope, the endoscope comprising an image capturing device, the image processing device comprising a processing unit operationally connectable to the image capturing device, wherein the processing unit is configured to:
obtain a stream of images captured by the image capturing device of the endoscope;
process the stream of images to estimate locations of a lumen in the stream of images, and
determine a quality measure of the endoscopic procedure based on the estimated locations of the lumen.

Consequently, by determining a location of a lumen and using the location for estimating a quality measure a simple method of determining a quality measure is provided.

The processing unit of the image processing device may be any processing unit, such as a central processing unit (CPU), a graphics processing unit (GPU), a microcontroller unit (MCU), a field-programmable gate array (FPGA), or any combination thereof. The processing unit may comprise one or more physical processors and/or may be combined by a plurality of individual processing units.

The image processing device may comprise a display, i.e. the image processing device may be integrated in a display unit. Alternatively / additionally, the image processing device may be operationally connectable to a separate display unit.

The processing unit may be configured to detect the lumen using an adaptive method such as a machine learning data architecture.

Alternatively, the processing unit may be configured to detect the lumen using a non-adaptive method relying on static rules. As an example, the processing unit may be configured to detect a lumen by using a method comprising: finding a set of connected pixels having an intensity value below a first threshold. The first threshold may be an absolute threshold or a threshold determined based on the average intensity in an image. A group of connected pixels may be a set of pixels, where each pixel shares an edge or a corner with at least one other pixel in the set of pixels. A lumen may be detected if a set of connected pixels is found having a size above a second threshold.

The quality measure may be presented to the user during the procedure e.g. on a display. Alternatively, the quality measure may be presented to the user after the procedure has been completed and / or stored in a memory unit connected to the processing unit of the image processing device.

In some embodiments, the processing unit is configured to estimate the location of the lumen by providing the stream of image to a machine learning data architecture trained to identify the location of lumens in endoscope images.

Consequently, an effective and reliable way of determining positions of lumens is provided.

The machine learning data architecture may be a supervised machine learning architecture. The machine learning data architecture may be trained by obtaining a training data set comprising endoscope images of different parts of different body cavities and for each endoscope image having a human operator identify a lumen (if present) e.g. a human operator may for each image firstly specify if a lumen can be seen in the image (if the image capturing device is pointing towards a wall of the body cavity no lumen is visible), and secondly the location of the lumen. If a quality measure for a particular type of endoscopic procedure is determined, then the training data set may comprise endoscope images from that particular type of endoscopic procedure. As an example, if the quality measure is for a colonoscopy procedure, then the training data set may comprise endoscope images from different colonoscopy procedures on different patients.

The location of the lumen may be a center of the lumen or a circumference of the lumen. The circumference of the lumen may be a circle e.g. the smallest circle that encompasses the lumen or the largest circle that can be fully contained within the lumen. Alternatively, the circumference may be a curve substantially arranged above the border of the lumen. The processing unit may implement the machine learning data architecture, i.e. the processing unit may provide the image to the machine learning data architecture by processing the image using the machine learning data architecture. Alternatively, the processing unit may provide the image, a parameterized version of the image, or a dimensionally reduced version of the image to another processing unit e.g. outside of the image processing device, where said another processing unit implements the machine learning data architecture.

In some embodiments, the machine learning data architecture is an artificial neural network such as a deep structured learning architecture.

The machine learning data architectures may be U-Net (reference: Ronneberger et el. "U-Net: Convolutional Networks for Biomedical Image Segmentation" (2015), arXiv:1505.04597 [cs.CV] or Mask R-CNN (reference: He et al. "Mask R-CNN" (2017), arXiv:1703.06870 [cs.CV].

In some embodiments the endoscopic procedure is a colonoscopy.

The location of the lumen in the image may indicate what area of the circumference of the colon is being investigated. Consequently, by using the estimated location of the lumen in the image for determining a quality measure, the quality measure may indicate if all parts of the colon have been sufficiently investigated.

In some embodiments, the processing unit is further configured to divide the circumference of the colon into a plurality of areas, based on the estimated location of the lumen in the stream of images estimate which area of the plurality of areas is being investigated, and for each area of the plurality of areas determine a quality measure.

Consequently, the medical professional may be provided with information not only specifying the overall quality of the colonoscopy, but detailed information specifying the quality of different areas of the colon. This may both be useful information for the medical professional during the procedure and after the procedure.

The circumference of the colon may be divided into equally large areas e.g. four equally large areas or 8 equally large areas. As an example, the areas may be upper left, upper right, lower left, and lower right.

In some embodiments, each area of the plurality of areas corresponds to an image zone of the stream of images and wherein the processing unit is configured to estimate that a particular area of the plurality of areas is being investigated if the estimated location of the lumen is arranged within the image zone of the particular area.

Consequently, a simple method of estimating which area of the circumference is being investigated is provided.

The image zones may have an equal size. The image zones may be centered around the center of image. The image zone may be arranged rotationally symmetric around the center of the image. Each image zone may have an opposing image zone with the same shape but only rotated 180 degrees around the center of the image.

In the event the lumen is located in more than one image zone, it may be decided that the lumen is present in the image zone where most of the lumen is present. However, it may also be determined that the lumen is only present in an image zone if the entire lumen is present in the image zone or at least a percentage of the lumen above a threshold.

In some embodiments, the processing unit is configured to, for one or more images of the stream of images where a lumen is not found, estimate which area of the plurality of areas is being investigated based on a previous image in the stream of images where a lumen if found and / or a subsequent image where a lumen is found.

Consequently, by using information from a previous image or subsequent image (where a lumen is visible), it may be estimated even for images where the lumen cannot be seen which area of the circumference of the colon is being investigated.

In some embodiments, the processing unit is further configured to divide the colonoscopy procedure into a plurality of parts and for each part estimate a quality measure.

Consequently, it may be secured that an efficient examination is being performed along the entire length of the colon.

In some embodiments, each part corresponds to a section of the colon having a predetermined length, and wherein the processing unit is configured to process the stream of images to estimate in which section the endoscope is located.

Consequently, it may be secured that an efficient examination is being performed along the entire length of the colon.

The length of each section may be 5cm, 10cm or 15cm. Each section may correspond to specific anatomical positions or be defined with respect to a specific anatomic position. Examples of specific anatomical positions are: the cecum, the anus, and the entrance to the appendix.

However, the location of each section may also be defined relative to a selected point e.g. the initial position of the colonoscope.

In some embodiments, the processing unit is configured to process the stream of images to estimate the withdrawal speed of the endoscope and use the estimated withdrawal speed to estimate in which section the endoscope is located.

Consequently, a simple method of determined the location of the endoscope is provided.

This may be especially advantageous when the location of each section is defined relative to a selected point e.g. the initial position of the colonoscope.

In some embodiments, the processing unit is configured to process the stream of images to determine a parameter value related to the sharpness of the images and estimate the withdrawal speed based on the parameter value.

The sharpness of an image is dependent on the speed of the endoscope. Specifically, if the endoscope is moving fast the resulting images will become less sharp than if the endoscope is moving slow.

Consequently, by determining the sharpness of images a simple way of estimating the withdrawal speed is provided.

The stream of images may be processed in the spatial domain and / or frequency domain to determine a parameter value related to the sharpness of the images. In the frequency domain a value may be determined specifying the percentage of energy in the image above a particular set of frequencies. A high value will be indicative of a sharp image and a low value will be indicative of a unsharp / blurry image. Alternatively, a set of frequencies may be found containing a particular amount of the energy in the image. Again, a high value will be indicative of a sharp image and a low value will be indicative of a unsharp / blurry image.

In the spatial domain the average change in intensity between neighboring pixels may be determined. A high value will be indicative of a sharp image and a low value will be indicative of a unsharp / blurry image.

The relationship between the parameter value and the withdrawal speed is dependent on a number of factors including the lightning, the optical system, the tissue type etc. In practice, it may be experimentally determined. A function may be found describing the relationship. Alternatively, a look-up table may be stored specifying the relationship.

In some embodiments, the processing unit is configured to estimate the withdrawal speed of the endoscope by providing the stream of images to a machine learning data architecture trained to estimate the withdrawal speed of an endoscope during a colonoscopy procedure based on endoscope images.

Consequently, an effective way of determining the withdrawal speed is provided.

The machine learning data architecture may be a supervised machine learning architecture. The machine learning data architecture may be trained by obtaining a training data set comprising a plurality of streams of endoscope images obtained from different colonoscopies, where for each stream of endoscope image the withdrawal speed is provided. In practice, when creating the training data set, the withdrawal speed may be measured using an extra measurement system. As an example, the endoscope may be provided with accelerometers enabling the withdrawal speed to be estimated. Alternatively, the withdrawal speed may be estimated by providing the insertion tube of the endoscope with markings e.g. a marking every 5 cm. The withdrawal speed may then be found by having another imaging system monitoring the anus of the patient and measuring the time it takes for a new marking to become visible. As an example, if the spacing between markings are 5cm and the time it takes for a new marking to become visible is 20 seconds, then the withdrawal speed is estimated to be 0.25cm/second. The extra measurement system is only needed for training, i.e. the extra measurement system is not needed when using the machine learning data structure to estimate the withdrawal speed.

In some embodiments, the machine learning data architecture is an artificial neural network such as a deep structured learning architecture.

The machine learning data architectures may be U-Net (reference: Ronneberger et el. "U-Net: Convolutional Networks for Biomedical Image Segmentation" (2015), arXiv:1505.04597 [cs.CV] or Mask R-CNN (reference: He et al. "Mask R-CNN" (2017), arXiv:1703.06870 [cs.CV].

According to a second aspect, the present disclosure relates to a display unit for displaying images obtained by an image capturing device of an endoscope, wherein the display unit comprises an image processing device as disclosed in relation to the first asepct.

According to a third aspect, the present disclosure relates to an endoscope system comprising an endoscope and an image processing device as disclosed in relation to the first aspect of the present disclosure,
wherein the endoscope has an image capturing device and the processing unit of the image processing device is operationally connectable to the image capturing device of the endoscope.

In some embodiments, the image processing device forms part of a display unit as disclosed in relation to the second aspect of the disclosure.

According to a fourth aspect, the present disclosure relates to a method for estimating a quality measure of a an endoscopic procedure performed using an endoscope, the endoscope comprising an image capturing device, wherein the method comprises:
obtain a stream of images captured by the image capturing device of the endoscope;
process the stream of images to estimate locations of a lumen in the stream of images, and
determine a quality measure of the endoscopic procedure based on the estimated locations of the lumen.

According to a fifth aspect, the present disclosure relates to a computer program product comprising program code means adapted to cause a data processing system to perform the steps of the method as disclosed in relation to the fourth aspect of the present disclosure, when said program code means are executed on the data processing system.

In some embodiments, said computer program product comprises a non-transitory computer-readable medium having stored thereon the program code means.

The different aspects of the present disclosure can be implemented in different ways including image processing devices, display units, endoscope systems, methods for estimating a quality measure, and computer program products described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependent claims. Furthermore, it will be appreciated that embodiments described in connection with one of the aspects described herein may equally be applied to the other aspects.

### Brief description of the drawings

The above and/or additional objects, features and advantages of the present disclosure, will be further elucidated by the following illustrative and non-limiting detailed description of embodiments of the present disclosure, with reference to the appended drawings, wherein:
Fig. 1 shows an example of an endoscope.
Fig. 2 shows an example of a display unit that can be connected to the endoscope shown in Fig. 1.
Fig. 3 shows a schematic drawing of an image processing device according to an embodiment of the disclosure.
Fig. 4 shows a flowchart of a method for estimating a quality measure of an endoscopic procedure according to an embodiment of the disclosure.
Figs. 5a-e show schematically endoscope views.
Fig. 6 illustrate how a machine learning data architecture may be trained to estimate locations of one or more lumens in endoscope images.
Fig. 7 illustrates how one or more lumen may be identified in endoscope images according to an embodiment of the disclosure.
Fig. 8 shows a flowchart of a method 800 for estimating quality measures of a coloscopy procedure performed using an endoscope, the endoscope comprising an image capturing device.

### Detailed description

In the following description, reference is made to the accompanying figures, which show by way of illustration how the embodiments of the present disclosure may be practiced.

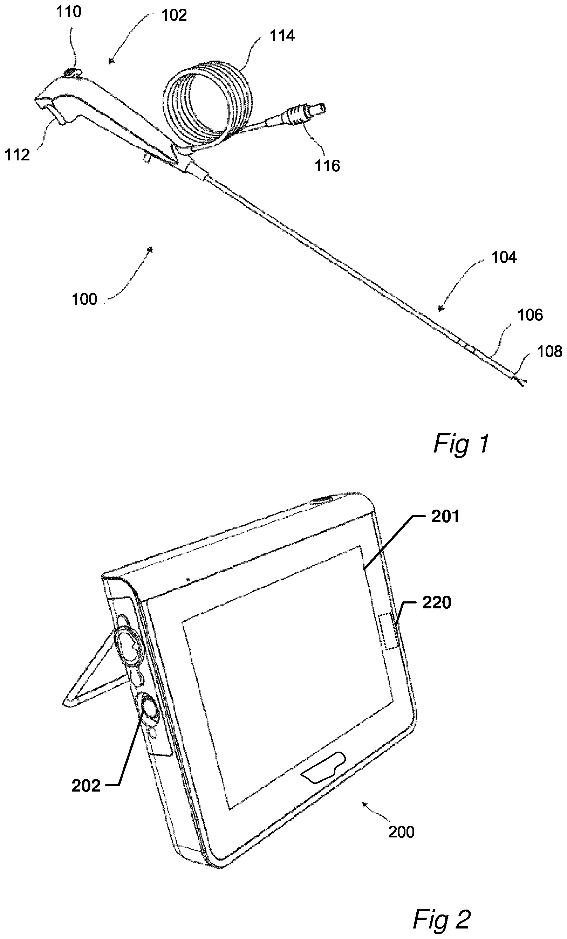

Fig. 1 illustrates an example of an endoscope 100. This endoscope may be adapted for single-use. The endoscope 100 is provided with a handle 102 attached to an insertion tube 104 provided with a bending section 106. The endoscope 100 may also be designed without a bending section 106. The illustrated endoscope 100 is a flexible endoscope with a flexible insertion tube 104, but the principle of the disclosure can also be used with any type of endoscope. Some embodiments of the disclosure relate to colonoscopy procedures. Such procedures are typically performed using a colonoscope.

The insertion tube 104 as well as the bending section 106 may be provided with one or several working channels such that instruments, such as a gripping device or a catheter, may extend from the tip and be inserted into a human body via the endoscope. One or several exit holes of the one or several channels may be provided in a tip part 108 of the endoscope 100. In addition to the exit holes, a camera sensor, such as a CMOS sensor or any other image capturing device, as well as one or several light sources, such as light emitting diodes (LEDs), fiber, or any other light emitting devices, may be placed in the tip part 108. By having the camera sensor and the light sources and a display unit 200, illustrated in Fig. 2, configured to display images based on image data captured by the camera sensor, an operator is able to see and analyse an inside of the human body in order to for instance localize a position for taking a sample. In addition, the operator will be able to control the instrument in a precise manner due to the provided visual feedback. Further, since some diseases or health issues may result in a shift in natural colours or other visual symptoms, the operator is provided with valuable input for making a diagnosis based on the image data provided via the image capturing device and the display unit 200. The display unit comprises a display 201, a processing unit 220 (only schematically shown), and a connection port 202 for operationally connecting the endoscope to the processing unit 220. The connection port 202 may further be used to provide power to the endoscope.

In order to make it possible for the operator to direct the camera sensor such that different field of views can be achieved, the endoscope has a bending section 106 that can be bent in different directions with respect to the insertion tube 104. The bending section 106 may be controlled by the operator by using a knob 110 placed on the handle 102. The handle 102 illustrated in Fig. 1 is designed such that the knob 110 is controlled by a thumb of the operator, but other designs are also possible. In order to control a gripping device or other device provided via a working channel a push button 112 may be used. The handle 102 illustrated in Fig. 1 is designed such that a index finger of the operator is used for controlling the gripping device, but other designs are also possible.

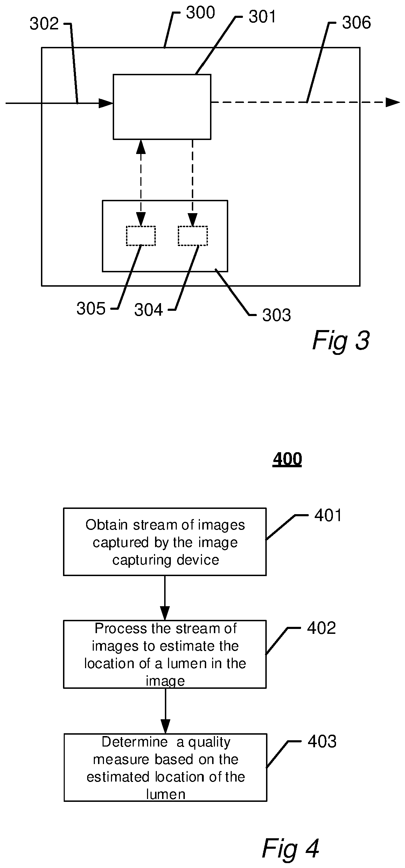

Fig. 3 show a schematic drawing of an image processing device 300 for estimating a quality measure of an endoscopic procedure performed using an endoscope (not shown) according to an embodiment of the disclosure. The endoscope comprises an image capturing device. The image processing device 300 comprises a processing unit 301 operationally connectable 302 to the image capturing device. The processing unit 301 is configured to obtain a stream of images captured by the image capturing device of the endoscope. The processing unit may be connectable to the image capturing device, e.g. by wire. As an example, the image processing device may comprise a connection port for operationally connecting the endoscope to the processing unit 301. The connection port may further be used to provide power to the endoscope. Alternatively, the processing unit 301 may be wireless connectable to the image capturing device e.g. the image processing device 300 may comprise a wireless communication unit (not shown) configured to receive images from the image capturing device. The processing unit 301 is further configured to process the stream of images to estimate the location of a lumen in the stream of images, and determine a quality measure 304 of the endoscopic procedure based on the estimated location of the lumen. The processing unit may be configured to estimate the location of the lumen by processing the stream of images using a machine learning data architecture 305 trained to identify the location of lumens in endoscope images. As an example, the machine learning data architecture may be stored in a memory unit 303 of the image processing device. The quality measure 304 may be stored in the memory unit 303. Alternatively / additionally, the quality measure may be provided 306 to a display (not) shown to be displayed to an operator, possibly together with endoscope images.

Fig. 4 shows a flowchart of a method 400 for estimating a quality measure of an endoscopic procedure performed using an endoscope, the endoscope comprising an image capturing device. In step 401, a stream of images captured by the image capturing device of the endoscope is obtained. Then in step 402, the stream of images is processed to estimate locations of a lumen in the stream of images. Then in step 403, a quality measure is determined of the endoscopic procedure based on the estimated locations of the lumen.

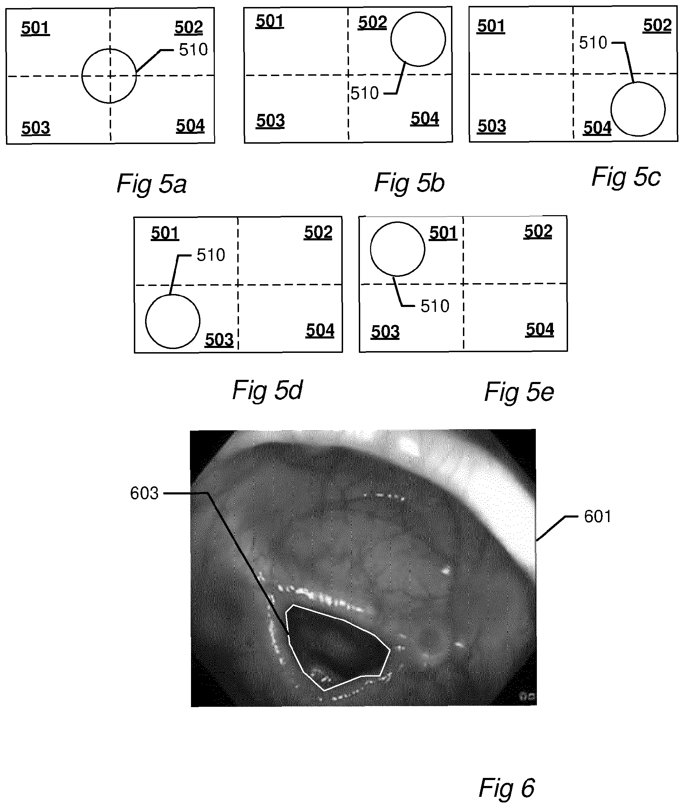

Fig. 5a-e show schematically images captured by an image capturing device of an endoscope during a colonoscopy and provided to the processing unit 301 of the image processing device 300 shown in Fig. 3 according to an embodiment of the present disclosure. In Fig. 5a is the lumen 510 located centrally. This is how an image may look when the endoscope is being withdrawn without investigating any particular area of the circumference of the colon. In Fig. 5b is the lumen 510 located in the upper right corner of the image. This is how an image may look when the lower left part of the circumference of the colon is being investigated. In Fig. 5c is the lumen 510 located in the lower right corner of the image. This is how an image may look when the upper left part of the circumference of the colon is being investigated. In Fig. 5d is the lumen 510 located in the lower left corner of the image. This is how an image may look when the upper right part of the circumference of the colon is being investigated. In Fig. 5e is the lumen 510 located in the upper left corner of the image. This is how an image may look when the lower right part of the circumference of the colon is being investigated.

The image processing device 300 of Fig. 3 may be configured to determine a quality measure of a colonoscopy. The processing unit 301 may be further configured to divide the circumference of the colon into a plurality of areas, and based on the estimated location of the lumen in the stream of images estimate which area of the plurality of areas is being investigated, and for each area of the plurality of areas determine a quality measure. The circumference of the colon may be divided into equally large areas e.g. four equally large areas or 8 equally large areas. As an example, the areas may be upper left, upper right, lower left, and lower right. Each area of the plurality of areas may corresponds to an image zone of the stream of images and wherein the processing unit is configured to estimate that a particular area of the plurality of areas is being investigated if the estimated location of the lumen is arranged within the image zone of the particular area.

In Figs. 5a-e the circumference of the colon is divided into 4 equally large areas. The areas are upper left, upper right, lower left, and lower right. Each area of the plurality of areas corresponds to an image zone 501 502 503 504 of the stream of images. In particular, the area upper left of the circumference corresponds to the image zone 504, the area upper right of the circumference corresponds to the image zone 503, the area lower left of the circumference corresponds to the image zone 502, and the area lower right of the circumference corresponds to the image zone 501. The processing unit 301 may be configured to estimate that a particular area of the plurality of areas is being investigated if the estimated location of the lumen is arranged within the image zone of the particular area. Thus, if the lumen 510 is located in the image zone 501 (see Fig. 5e) then it will be determined that lower right area is being investigated, if the lumen 510 is located in the image zone 502 (see Fig. 5b) then it will be determined that lower left area is being investigated, if the lumen 510 is located in the image zone 503 (see Fig. 5d) then it will be determined that upper right area is being investigated, and if the lumen 510 is located in the image zone 504 (see Fig. 5c) then it will be determined that the upper left area is being investigated. The quality measure for each area may be based on the amount of time spend on investigating the area e.g. the quality measure may be based on one or more thresholds. A first threshold may be used to determine the quality measure. Thus, if the medical professional uses more time than the first threshold on investigating an area, then a high quality measure may result. Additionally, a second first threshold may be used to determine the quality measure. Thus, if the medical professional uses more time than the second threshold but less than the first threshold on investigating an area, then a mediocre quality measure may result, and if the medical professional uses less time than the second threshold then a low quality measure may results.

Fig. 6 illustrates how a machine learning data architecture may be trained to estimate locations of a lumen in endoscope images. Shown is a single image 601 of a training data set. A typical training data set comprises endoscope images of different parts of the colon from the different procedures, i.e. endoscope images from a significant number of endoscope examinations. To train the machine learning data architecture a human operator indicates the location of the lumen 603. The location of the lumens is indicated by drawing up the circumference of the lumen 603.

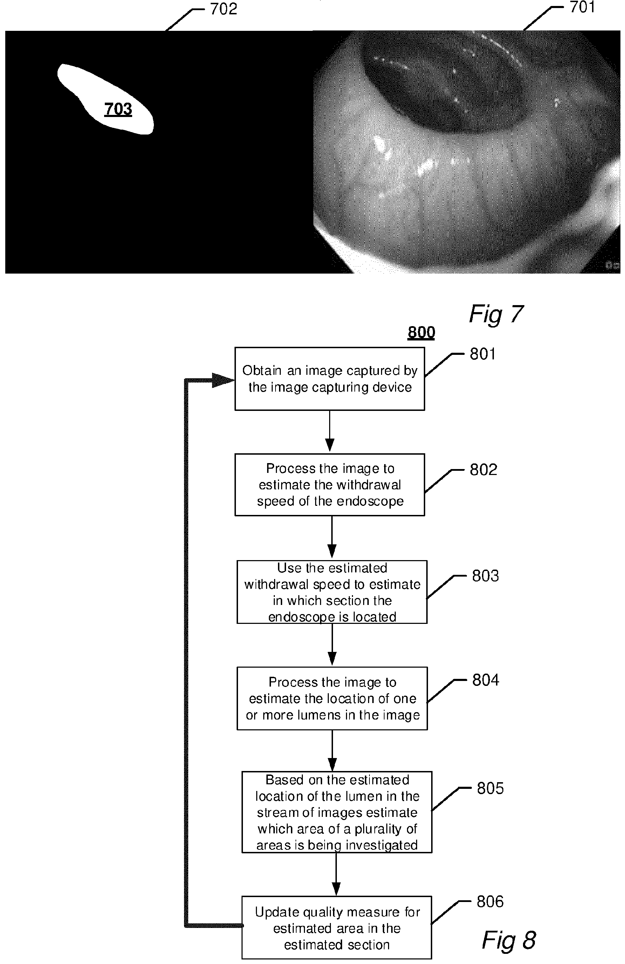

Fig. 7 illustrates how a lumen may be identified in endoscope images according to an embodiment of the disclosure. The lumen is identified using a machine learning data architecture trained to estimate the location of a lumen in endoscope images as disclosed in relation to Fig. 6. In Fig. 7 the input image 701 is shown to the right and the output from the machine learning data architecture 702 is shown to the left. As the machine learning data architecture has been trained to estimate the circumference of lumen, not only the location of the lumen 703 is estimated but also the circumference of the lumen 703.

Fig. 8 shows a flowchart of a method 800 for estimating quality measures of a coloscopy procedure performed using an endoscope, the endoscope comprising an image capturing device. In step 801, an image captured by the image capturing device of the endoscope is obtained. Then, in step 802 the obtained image is processed to estimate the withdrawal speed of the endoscope. Next, in step 803 the estimated withdrawal speed is used to estimate in which section of a plurality of section of the colon the endoscope is located. Each section of the plurality of sections may have a predetermined length e.g. each section may have a length of 5cm, 10cm or 15cm. Then in step 804 the image is processed to estimate the location of a lumen. Next, in step 805 it is estimated what area of a plurality of areas of the circumference of the colon is being investigated based on the estimated location of the lumen e.g. in the same way as disclosed in relation to Figs. 5a-e. Finally, the quality measure for the estimated area of the circumference of the colon for the estimated section is updated in step 806. As an example, if the quality measure for each area is based on the amount of time spend on investigating the area, then the quality measure for the estimated area of the circumference of the colon for the estimated section may be updated based on how often a new image is obtained e.g. if a new image is obtained with a frequency of 60Hz then the amount of time spend may be increased with 1/60 seconds. Finally, the method returns to step 801 where a new image is obtained. The frequency with which a new image is obtained may be the same as the frequency of the image capturing device but it may also be lower e.g. only every second or every fourth captured image may be processed to estimate a quality measure. The final output of the method after the colonoscopy has been completed is a quality measure for each area of the circumference of the colon for each section of the colon. Thus, if the colon is divided into 10 sections and the circumference of the colon is divided into 4 areas, then a total of 40 quality measures will be estimated. This will allow the medical professional not only to obtain an overall evaluation of the quality of the procedure, but a detailed map of the quality of the different parts of the procedure. This, may also assist the medical professional in performing a high quality procedure if the quality measures is presented to the medical professional during the colonoscopy procedure, as the medical professional may wait with withdrawing the endoscope from the current section of the colon until all areas of the circumference of the current section have been properly investigated. It should be noted that the steps of the method may be performed in different order e.g. steps 804 and 805 may be performed before steps 802 and 803.

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. An image processing device for estimating a quality measure of a endoscopic procedure performed using an endoscope, the endoscope comprising an image capturing device, the image processing device comprising a processing unit operationally connectable to the image capturing device, wherein the processing unit is configured to:
obtain a stream of images captured by the image capturing device of the endoscope;
process the stream of images to estimate locations of a lumen in the stream of images, and
determine a quality measure of the endoscopic procedure based on the estimated locations of the lumen.

2. An image processing device according to claim 1, wherein the processing unit is configured to estimate the location of the lumen by providing the stream of image to a machine learning data architecture trained to identify the location of lumens in endoscope images.

3. An image processing device according to claims 1 or 2, wherein the endoscopic procedure is a colonoscopy.

4. An image processing device according to claim 3, wherein the processing unit is further configured to divide the circumference of the colon into a plurality of areas, based on the estimated location of the lumen in the stream of images estimate which area of the plurality of areas is being investigated, and for each area of the plurality of areas determine a quality measure.

5. An image processing device according to claim 4, wherein each area of the plurality of areas corresponds to an image zone of the stream of images and wherein the processing unit is configured to estimate that a particular area of the plurality of areas is being investigated if the estimated location of the lumen is arranged within the image zone the particular area.

6. An image processing device according to any one of claims 1 to 5, wherein the processing unit is further configured to divide the colonoscopy procedure into a plurality of parts and for each part estimate a quality measure.

7. An image processing device according to claim 6, wherein each part corresponds to a section of the colon having a predetermined length, and wherein the processing unit is configured to process the stream of images to estimate in which section the endoscope is located.

8. An image processing device according to claim 6, wherein the processing unit is configured to process the stream of images to estimate the withdrawal speed of the endoscope and use the estimated withdrawal speed to estimate in which section the endoscope is located.

9. An image processing device according to claim 8, wherein the processing unit is configured to process the stream of images to determine a parameter value related to the sharpness of the images and estimate the withdrawal speed based on the parameter value

10. An image processing device according to claim 8, wherein the processing unit is configured to estimate the withdrawal speed of the endoscope by providing the stream of images to a machine learning data architecture trained to estimate the withdrawal speed of an endoscope during a colonoscopy procedure based on endoscope images.

11. A display unit for displaying images obtained by an image capturing device of an endoscope, wherein the display unit comprises an image processing device according to any one of claims 1 to 10.

12. An endoscope system comprising an endoscope and an image processing device according to any one of claims 1 to 10, wherein the endoscope has an image capturing device and the processing unit of the image processing device is operationally connectable to the image capturing device of the endoscope.

13. A method for estimating a quality measure of an endoscopic procedure performed using an endoscope, the endoscope comprising an image capturing device, wherein the method comprises:
obtain a stream of images captured by the image capturing device of the endoscope;
process the stream of images to estimate locations of a lumen in the stream of images, and
determine a quality measure of the endoscopic procedure based on the estimated locations of the lumen.

14. A computer program product comprising program code means adapted to cause a data processing system to perform the steps of the method according to claim 13, when said program code means are executed on the data processing system.

15. A computer program product according to claim 14, wherein said computer program product comprises a non-transitory computer-readable medium having stored thereon the program code means.
